# EUROPEAN PATENT APPLICATION

(11) **EP 2 044 973 A1**
(43) Date of publication of application: **08.04.2009**
(21) Application number: 07460023.0
(22) Date of filing: 03.10.2007
(51) Int. Cl.: A61N 5/06, H05B 33/08

(54) **Light-emitting diode (LED) light therapy device**

(71) Applicant: Aracaria B.V., 1017 JP Amsterdam (NL)
(72) Inventor: Vibor, Mulic, 00-730 Warszawa (PL)
(74) Representative: Viering, Jentschura & Partner

(57) **Abstract**

The light-emitting diode (LED) light therapy device is used especially in the preventive treatment, rehabilitation and the treatment of many diseases.

The light-emitting diode (LED) light therapy device is characteristic by its feature of being equipped with a matrix board (1) and a control system board (2), connected to a keypad (3), while the matrix board (1) comprises red light wavelength LED diodes (4) and blue light wavelength LED diodes (5), and the control system board (2) is equipped with a pre-programmed processor (11), which controls the LED diodes within the red light wavelength range (4) and the blue light wavelength range (5) via the keypad (3), a buzzer (9) and indicator lamps (10).

## Description

The subject of the invention is a light-emitting diode (LED) light therapy device to be used especially in the preventive treatment, rehabilitation and the treatment of many diseases.

The therapeutic effect of LED light exposure is produced by the LED radiation modulation of suitable frequencies. These frequencies produce resonance with the natural frequencies of organism cells, stimulate those cells and produce temporary depolarisation thereof. For different kinds of therapy, diversified values of the light modulating frequency ranging from 0 to 8000 Hz are used. The optimum pulsation frequency is dependent on the type of exposed tissues and the nature of disorders.

The light therapy efficacy is dependent on the organism's reaction to a radiation dose. The efficacy is determined by the Arndt-Schultz-Oshiro law, which says that a radiation dose between 0.1 and 16 J/cm² produces a positive reaction of an organism, while the maximum reaction occurs between 4 and 12 J/cm². Doses of 4 J/cm² are used most frequently, which corresponds to power density of 50 mW/cm². The light wavelength allowing light penetration into a human body is between 550 and 950 nm, while its optimum is about 700 nm.

There is a light therapy device known from the Polish P-372056 patent description, which consists of a control system and lighting points that are characterized by colour lighting points, favourably multicolour diodes, favourably RGB LED diodes around the light therapy location. The light therapy device has lighting points arranged at least in one row along the longitudinal axis sides of a bath, favourably a hydro-massage one. The light therapy device has lighting points arranged symmetrically in the shower cabin walls and ceiling.

The LED light therapy device, according to the invention, is characterized by its equipment comprising a matrix board and control system board connected to a keypad. The matrix board comprises light-emitting diodes (LEDs) in the red light wavelength range and the blue light wavelength range. It is equipped with a power supply socket, a battery socket and cables connecting the matrix board with the control system board. As LED diodes, diodes the structure of which is based on GaAlAs semiconductors generating electromagnetic waves within the red light wavelength range and InGaN/GaN semiconductors generating electromagnetic waves within the blue light wavelength range are used in the device, and diodes of the wavelength favourably of 625-680 nm and radiation angle of 10-30 degrees are used as the red light wavelength LEDs, while diodes of the wavelength of favourably 465-475 nm and radiation angle of 10-30 degrees are used as the blue light wavelength LEDs. The matrix board favourably comprises 108 LED diodes, whereas the quantitative ratio of the red light wavelength LEDs to the blue light wavelength LEDs (5) is 1:1. The red light wavelength LED diodes and the blue light wavelength LED diodes are alternately arranged on the matrix board in a chequered pattern. The control system board is equipped with a pre-programmed processor, which controls the LED diodes within the red light wavelength range, the blue light wavelength range, a buzzer and indicator lamps. The processor is controlled via a keypad equipped with an ON/OFF button and three buttons used to select the LED light exposure program within the red and blue light wavelength.

The solution under the invention allows a user to make local exposure of the affected areas of the body, finally resulting in a positive therapeutic and preventive reaction in the whole human organism by accelerating its healing, reconstructing the disturbed functions of the organism and increasing its immunity. The LED diodes applied in the device under the invention emit light of the most physiological, therapeutic effect. The combination of the red light wavelengths and blue light wavelengths with the pulsatory emission thereof creates the best conditions for the treatment, preventive treatment and rehabilitation.

The subject of the invention is shown as an example of an assembled device in the following figures:

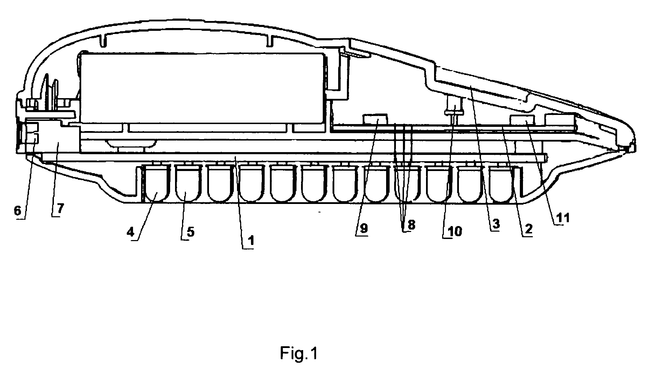

Fig. 1 shows a longitudinal section of the device,

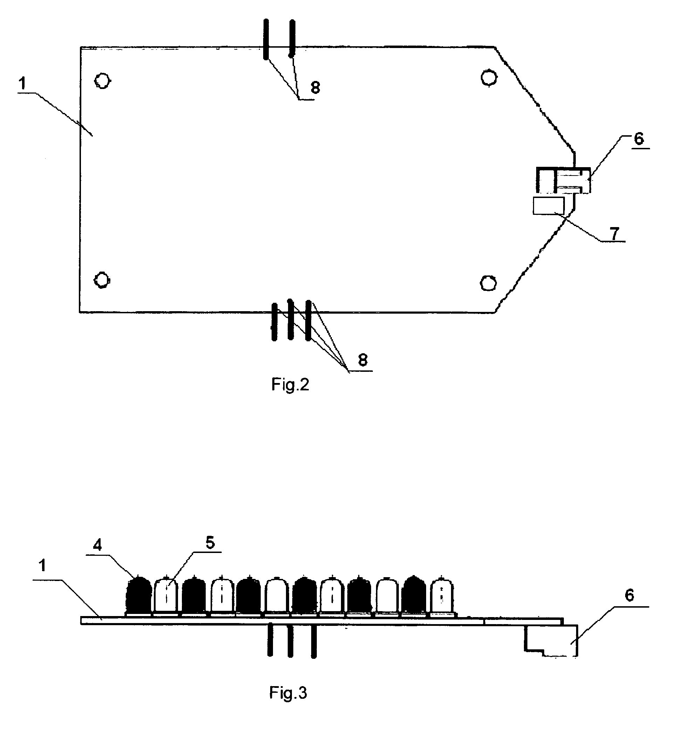

Fig. 2 shows the matrix board of the device in a top view,
Fig. 3 shows the matrix board of the device in a longitudinal section,

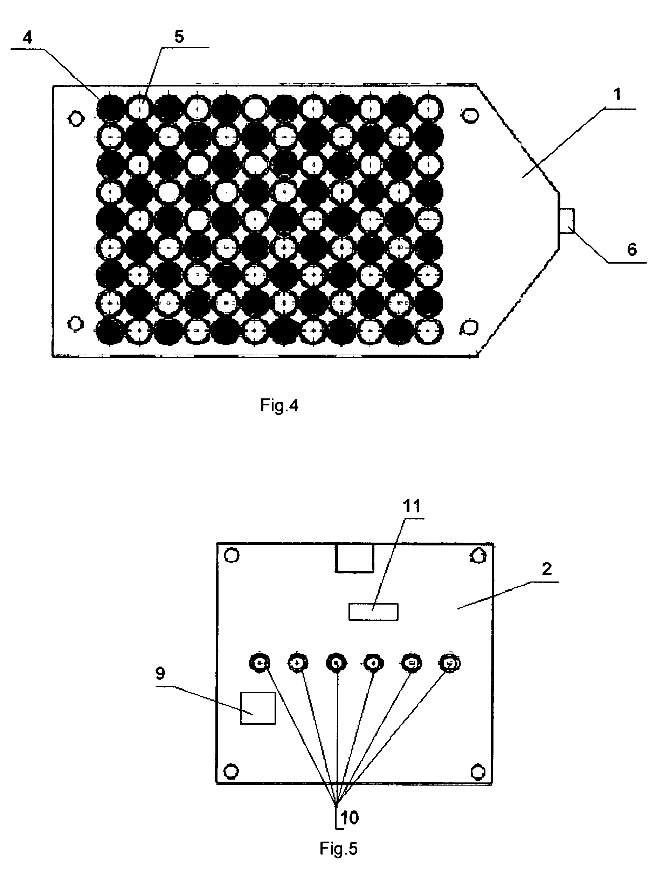

Fig. 4 shows the matrix board of the device in a bottom view,
Fig. 5 shows the control system board of the device in a top view,

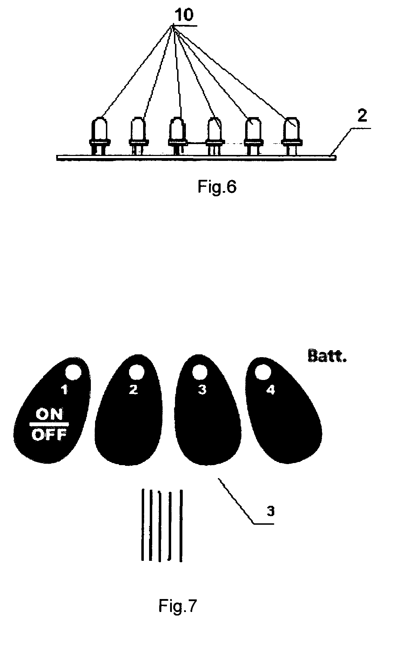

Fig. 6 shows the control system board of the device in a longitudinal section,
Fig. 7 shows a view of the keyboard of the device.

The LED light therapy device is equipped with a matrix board 1 and a control system board 2 connected to a keypad 3. The matrix board 1 comprises LED diodes within the range of the red light wavelength 4 and the blue light wavelength 5 and it is equipped with a power supply socket 6, a battery socket 7 and cables 8, which connect the matrix board 1 to the control system board 2. The matrix board 1 comprises 108 LED diodes, including 54 red light wavelength range LEDs 4 and 54 blue light wavelength range LEDs 5. The red light wavelength LED diodes 4 and the blue light wavelength LED diodes 5 are alternately arranged on the matrix board 1 in a chequered pattern. The control system board 2 is equipped with a pre-programmed processor 11, which controls the LED diodes within the red light wavelength range 4 and the blue light wavelength range 5, a buzzer 9 and indicator lamps (10). The processor 11 is controlled via a keypad 3 equipped with an ON/OFF button and three buttons used to select the LED light exposure program within the red 4 and blue 5 light wavelength range.

### Program 1.

The blue and red button on the keypad 3 is used to select the exposure mode of the device with the diodes emitting alternately blue light and red light (MODE 1). First, the LEDs emitting continuous blue light 5 for the amount of time specified in the program illuminate, then the LEDs emitting pulsating red light 4 of the pulsation frequency of 8000 Hz automatically illuminate for the amount of time specified in the program.

### Program 2.

The blue button on the keypad 3 is used to select the exposure mode of the device operation for a specific length of time with the diodes emitting a continuous blue light (MODE 2).

### Program 3.

The red button on the keypad 3 is used to select the exposure mode of the device operation with the diodes emitting a pulsing red light, with a pulsation frequency of 8000 Hz, for a length of time set in the program.

The exposure completes automatically in each of the programs after a programmed exposure time ends. The end of exposure and each instance of pressing the buttons on the keypad 3 of the device under the invention is indicated by an aural signal.

## Claims

1. The light-emitting diode (LED) light therapy device is characteristic by its feature of being equipped with a matrix board (1) and a control system board (2), connected to a keypad (3), while the matrix board (1) comprises red light wavelength LED diodes (4) and blue light wavelength LED diodes (5) and it is equipped with a power supply socket (6), a battery socket (7) and cables (8), which connect the matrix board (1) with the control system board (2) equipped with a pre-programmed processor (11) controlling the red light wavelength range LEDs (4) and the blue light wavelength range LEDs (5), a buzzer (9) and indicator lamps (10), while the processor (11) is controlled via the keypad (3) equipped with an ON/OFF button used to switch on/off the device and three buttons used to select a LED light exposure program within the red light wavelength range (4) and the blue light wavelength range (5).

2. The device according to claim No. 1 is characteristic by the LED diodes, the structure of which is based on GaAlAs semiconductors generating electromagnetic waves within the red light wavelength range and InGaN/GaN semiconductors generating electromagnetic waves within the blue light wavelength range are used in the device that are favourably used.

3. The device according to claim No. 1 is characteristic by the LEDs favourably of the wavelength of 625-680 nm and the radiation angle of 10-30 degrees that are used as the red light wavelength range LEDs (4).

4. The device according to claim No. 1 is characteristic by the LEDs favourably of the light wavelength of 465-475 nm and the radiation angle of 10-30 degrees that are used as the blue light wavelength range LEDs (5).

5. The device according to claim No. 1 is characteristic by the matrix board (1) favourably comprising 108 light-emitting diodes (LEDs), whereas the quantitative ratio of the red light wavelength range LEDs to the blue light wavelength range LEDs (5) is 1:1.

6. The device according to claim No. 1 is characteristic by the red light wavelength range LED diodes (4) and the blue light wavelength range LED diodes (5) that are alternately arranged on the matrix board (1) in a chequered pattern.
